# EUROPÄISCHE PATENTANMELDUNG

(11) **EP 0 943 293 A1**
(43) Veröffentlichungstag der Anmeldung: **22.09.1999**
(21) Anmeldenummer: 99105275.4
(22) Anmeldetag: 15.03.1999
(51) Int. Cl.: A61B 17/28

(54) **Vorrichtung zur Betätigung eines Greifmechanismus an einer endoskopischen Sonde**

(30) Priorität: 19.03.1998 DE 19812101
(71) Anmelder: B. BRAUN MELSUNGEN AG, 34212 Melsungen (DE)
(72) Erfinder: Frimberger, Eckart, Dr., 80804 München (DE)
(74) Vertreter: Körber, Wolfhart, Dr.

(57) **Zusammenfassung**

Eine Vorrichtung zur endoskopischen Entnahme von Gewebeproben (Biopsie) weist eine Sonde (1) mit einem Greifmechanismus (3) zur Entfernung einer Gewebeprobe, eine Betätigungseinheit (10) mit Aufnahmeelementen (11, 12) zur lösbaren Arretierung eines Griffteils des Entnahmewerkzeugs (1), und eine Antriebseinheit zur Betätigung des Greifmechanismus (3) der an der Betätigungseinheit (10) arretierten Sonde (1) auf. Die Vorrichtung ermöglicht dem Endoskopiker eine vereinfachte und verbesserte Handhabung der Entnahme von Gewebeproben. Insbesondere kann die Biopsie ohne assistierende Hilfsperson durchgeführt werden.

## Beschreibung

Die Erfindung betrifft eine Vorrichtung zur Betätigung eines Greifmechanismus an einer Sonde, vorzugsweise zur endoskopischen Entnahme von Gewebeproben oder Fremdkörpern, aufweisend eine Hohlsonde mit einem Greifmechanismus, einer Betätigungseinheit mit Aufnahmeelementen zur lösbaren Arretierung an einem Griffteil der Sonde und einer Antriebseinheit in der Betätigungseinheit zur kontrollierten Bewegung eines in der Hohlsonde angeordneten Zugseiles zur Betätigung des Greifmechanismus.

Im Rahmen der endoskopischen Diagnostik werden häufig aus verschiedenen Organen, beispielsweise dem Magen-Darm-Trakt mit Hilfe geeigneter Instrumente Gewebsproben entnommen (Biopsie). Die dafür verwendete Sonde wird dabei in den Instrumentierkanal des Endoskops eingeführt und kann mittels eines durch den Instrumentierkanal geführten Bowdenzuges von außerhalb des Körpers des Patienten betätigt werden. Ein vorzugsweise dafür eingesetztes Instrument ist die Biopsie-Zange im wesentlichen bestehend aus einem Bowdenzug mit Griff und einem Zangenmaul. Durch Griffbetätigung kann das Zangenmaul geöffnet und geschlossen werden, wodurch ein Gewebspartikel vom Gewebe entfernt und festgehalten werden kann. Der Endoskopiker führt die Biopsie-Zange durch den Instrumentierkanal des Endoskops an den Einsatzort im Körper, die assistierende Schwester betätigt die Zange zur Gewebsentnahme (Biopsie). Nach der Biopsie zieht der Endoskopiker die Zange aus dem Endoskop zurück, die Schwester hält mit der einen Hand den Griff der Biopsie-Zange, führt mit der anderen Hand das Zangenmaul in ein Probengläschen, wo die Probe durch Schütteln der Zange entfernt wird und in das mit konservierender Flüssigkeit gefüllte Gläschen fällt.

Bekannte Vorrichtungen zur endoskopischen Entnahme von Gewebeproben lassen sich somit nicht durch eine Person alleine betätigen, sondern erfordern zur Handhabung neben dem Endoskopiker eine assistierende Krankenschwester/Krankenpfleger.

Ein weiterer Nachteil der bekannten Vorrichtungen zur endoskopischen Entnahme von Gewebeproben ist die schlechte Einstellbarkeit der Betätigungskraft des Zangenmauls des Greifmechanismus. Eine zu geringe Kraft kann bei derbem Gewebe zur Probenentnahme nicht ausreichend sein, andererseits führt eine zu große Betätigungskraft zu stärkerem Verschleiß der sehr kleinen beweglichen Teile der Sonde.

Der Erfindung liegt die Aufgabe zugrunde, eine Vorrichtung der eingangs genannten Art zu schaffen, die eine einfache Handhabung, das heißt Inbetriebnahme und Ablage der Sonde nach Benutzung durch eine einzelne Person, insbesondere den behandelnden Arzt ohne Assistenz, gegebenenfalls auch einen schnellen Austausch der Sonde gegen eine andere Sonde ermöglicht.

Gelöst wird diese Aufgabe durch eine Vorrichtung nach Anspruch 1.

Dadurch läßt sich die Sonde schnell und einfach mit der Antriebseinheit koppeln und wieder entfernen und es ist möglich, die Vorspannung und Betätigung des Zugseiles der Sonde durch die Antriebseinheit genau zu kontrollieren. Zweckmäßige Ausgestaltungen der Erfindung sind in den Unteransprüchen 2 bis 8 gekennzeichnet.

Die Probenentnahme erfolgt mit der an der Betätigungseinheit arretierten Sonde durch die Antriebseinheit. Sie kann daher mittels eines geeigneten Betätigungsmechanismus, beispielsweise eines Fußpedals durch den Endoskopiker selbst ausgeführt werden, so daß dafür eine assistierende Krankenschwester nicht benötigt wird. Außerdem läßt sich die Zugriffskraft des Greifmechanismus definiert einstellen.

Der Griffbereich des Entnahmewerkzeugs weist vorzugsweise zwei Eingriffsnuten zur Aufnahme durch die Aufnahmeelemente der Betätigungseinheit auf, wobei eines der Aufnahmelemente mechanisch mit dem Zugseil des Bowdenzuges der Sonde verbunden und das andere gegenüber diesem beweglich angeordnet ist. So kann die Bewegung von den Aufnahmeelementen der Betätigungseinheit auf das Zugseil und den Greifmechanismus übertragen werden. Vorzugsweise können an dem Griffbereich des Entnahmewerkzeugs mehrere voneinander beabstandete Nuten ausgebildet sein, so daß ein Einrasten in verschiedenen Positionen möglich ist, die beispielsweise durch eine Krümmung des aus Zugseil und Mantelsonde bestehenden Bowdenzuges hervorgerufen werden.

Die Betätigungseinheit ist vorzugsweise drehbar und höhenverstellbar an einer Halterung befestigt, so daß eine optimale Anordnung zum Patienten ermöglicht wird. Außerdem wird durch die Drehbarkeit der Betätigungseinheit ein Abknicken des Bowdenzuges verhindert.

Die Aufnahmeelemente der Betätigungseinheit sind zueinander beweglich angeordnet. Wenn die Sonde in die Aufnahmeelemente einrastet, wird das Aufnahmeelement vorzugsweise so verfahren, daß eine definierte Vorspannung zum Schließen des Greifmechanismus auf das Zugseil ausgeübt wird. Der Endoskopiker kann dann die geschlossene Zange problemlos durch den Instrumentierkanal des Endoskops einführen. Die Vorspannung kann geeignet, beispielsweise zu etwa 30 N eingestellt werden. Ein Druckschalter bzw. eine Lichtschranke kann als Detektor zur Erfassung des Einrastens der Sonde vorgesehen sein.

Die Sonde läßt sich vorzugsweise in mindestens drei unterschiedliche Zustände steuern. Eine Grundposition mit einer Vorspannung von beispielsweise 10-60 N, eine geöffnete Position des Greifwerkzeugs und eine Position mit verschärftem Zugriff von beispielsweise 60-120 N Kraft oder mehr bei besonders hartem Gewebe.

Der Antrieb kann elektromagnetisch, insbesondere durch einen Elektromotor oder hydraulisch oder durch Preßluft erfolgen. Die Kraftübertragung von der Antriebseinheit kann durch Bowdenzüge oder hydraulisch erfolgen. Die Antriebseinheit kann beispielsweise über Fußpedale betätigbar sein. Dies ermöglicht dem Endoskopiker, daß er die Hände frei hat.

Eine besonders zweckmäßige Weiterbildung der Erfindung ist in den Ansprüchen 9 bis 12 gekennzeichnet. Dieses Führungsrohr erlaubt die Ablage der Sonde nach Benutzung ohne fremde Hilfe. Dieses Führungsrohr kann aus Kunststoff ausgebildet und entfernbar, insbesondere als Wegwerfteil ausgebildet sein. Die Führung des Führungsrohrs in Zusammenwirken mit der Drehbarkeit des Betätigungsmechanismus erlaubt eine Schlaufenbildung der Sonde derart, daß eine Berührung und somit eventuelle Kontamination von anderen Teilen der Vorrichtung, insbesondere des Halterungsgestänges vermieden wird. Es ist daher nicht erforderlich, daß eine Krankenschwester die Entnahmevorrichtung nach erfolgter Gewebeentnahme in Empfang nimmt und auf einer geeigneten Ablage ablegt.

Die Erfindung wird im folgenden anhand von bevorzugten Ausführungsbeispielen unter Bezugnahme auf die Zeichnung näher erläutert, in der
- Figur 1: eine schematische Darstellung einer erfindungsgemäßen Sonde ist;
- Figur 2: eine schematische Darstellung einer erfindungsgemäßen Betätigungseinheit ist;
- Figur 3: eine Seitenansicht zur Illustration des Einrastmechanismus der Sonde an der Betätigungseinheit ist;
- Figur 4: eine Seitenansicht und eine Frontansicht eines Halteelements der erfindungsgemäßen Betätigungseinheit ist;
- Figur 5: eine schematische Seitenansicht zur Illustration eines Funktionsprinzips der erfindungsgemäßen Vorrichtung zur endoskopischen Entnahme von Gewebeproben ist;
- Figur 6: eine schematische Darstellung zur Erläuterung eines Funktionsprinzips der erfindungsgemäßen Sonde ist;
- Figur 7: eine schematische Darstellung eines abgewandelten Ausführungsbeipiels der erfindungsgemäßen Sonde ist;
- Figur 8: eine schematische Darstellung der erfindungsgemäßen Vorrichtung zur endoskopischen Entnahme von Gewebeproben ist, wobei ein abgewandeltes Ausführungsbeispiel der erfindungsgemäßen Betätigungseinheit gezeigt ist.; und
- Figur 9: das vordere Ende eines Ausführungsbeispiels der erfindungsgemäßen Sonde zeigt.

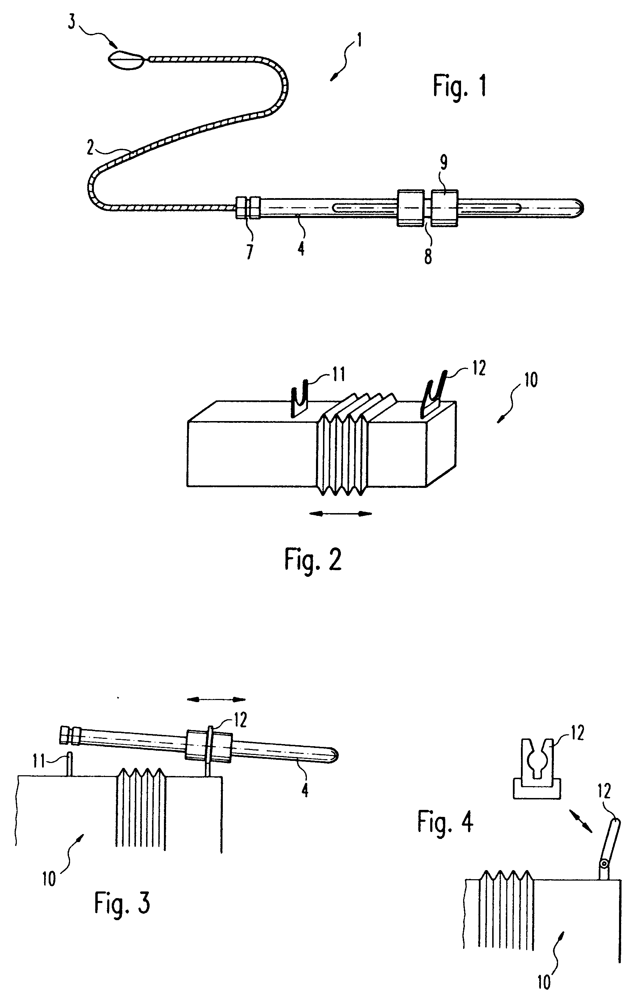

Figur 1 zeigt schematisch ein Ausführungsbeispiel der Sonde 1 der erfindungsgemäßen Vorrichtung zur endoskopischen Entnahme von Gewebeproben. Ein bewegbares Zangenmaul bildet einen Greifmechanismus 3 zum Entfernen und Festhalten eines Gewebspartikels. Der Greifmechanismus 3 ist über einen als Hohlsonde 2 ausgebildeten Bowdenzug mit einem Griffteil 4 verbunden. Das Zangenmaul läßt sich mit dem Bowdenzug 2 in den Instrumentierkanal des (nicht gezeigten), an sich bekannten Endoskops an die gewünschte Stelle bzw. das gewünschte Organ, wo die Gewebeentnahme durchgeführt werden soll, einführen. Das Griffteil 4 der Sonde 1 weist eine vordere Nut 7 und eine hintere Nut 8 auf. Die hintere Nut 8 in einem Verschiebeteil 9 ist mechanisch mit dem Zugseil des Bowdenzugs 2 gekoppelt, während die vordere Nut 7 in der Griffhülle ausgebildet ist, die gegenüber dem Zugseil frei beweglich und dem Mantelteil des Bowdenzuges 2 fixiert ist.

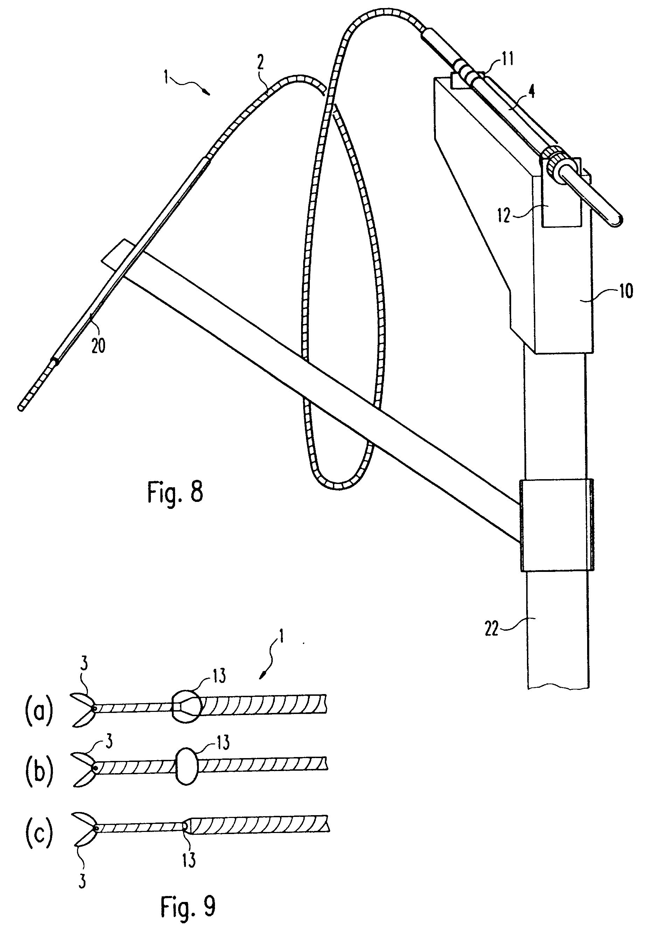

Figur 2 zeigt ein Ausführungsbeispiel der Betätigungseinheit der erfindungsgemäßen Vorrichtung zur endoskopischen Entnahme von Gewebeproben. Die gezeigte Betätigungseinheit besteht aus zwei zueinander bewegbaren quaderförmigen Gehäuseteilen, die über einen Faltenbalg miteinander verbunden sind. Das Gehäuse kann jedoch genauso einen zylindrischen Querschnitt haben oder eine andere geeignete Form aufweisen. Die Betätigungseinheit 10 weist zueinander bewegbare Aufnahmeelemente 11, 12 auf, in welche das Griffteil 4 der Sonde 1 mittels der Nuten 7, 8 lösbar arretiert werden kann. Während bei dem in Figur 2 gezeigten Ausführungsbeispiel die Halteelemente 11, 12 auf den kubusförmigen Gehäuseteilen angeordnet sind, ist bei dem in Figur 8 gezeigtem Ausführungsbeispiel ein Aufnahmeelement auf dem (hier in Form eines rechtwinkligen Dreiecks ausgebildeten) Gehäuse und das zweite mittels einer zylindrischen Stange gegenüber dem Gehäuse verfahrbar angeordnet. Vorzugsweise ist eines der Aufnahmeelemente 12 zur Erleichterung der Arretierung und zum Lösen des Griffteils der Sonde kippbar angeordnet, wie am besten in den Figuren 3 und 4 dargestellt ist.

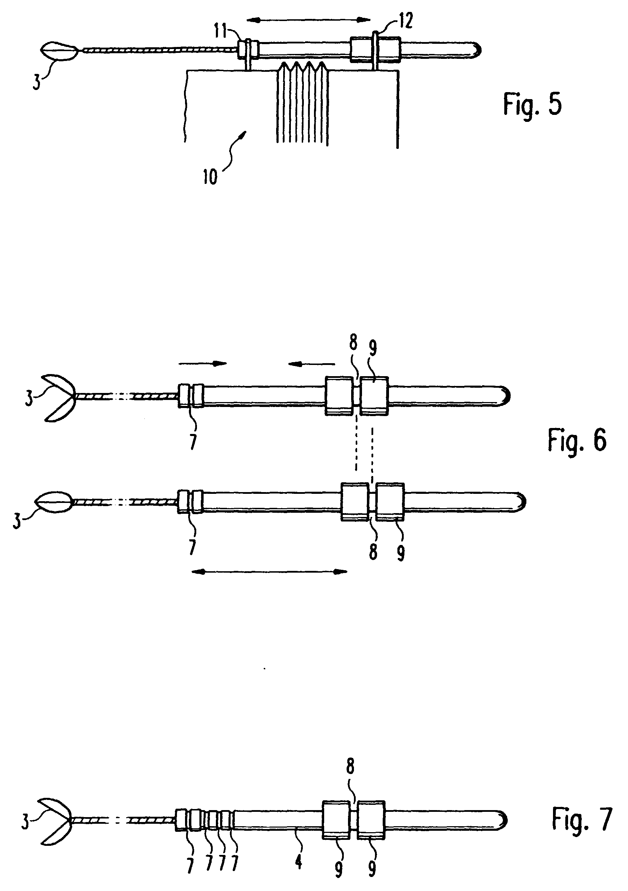

Eine mit der Betätigungseinheit verbundene (nicht gezeigte) Antriebseinheit verschiebt beim Einlegen des Griffteils in eines der Aufnahmeelemente elektronisch geregelt das andere Aufnahmeelement so, daß der Abstand der Aufnahmeelemente voneinander dem Abstand der Nuten 7, 8 des Griffteils 4, 9 der Sonde entspricht. Sobald das Entnahmewerkzeug 1 mit den Nuten 7, 8 in den Aufnahmeelementen 11, 12 der Betätigungseinheit 10 einrastet, wird dies von einem (nicht gezeigten) Detektor, beispielsweise einem Druckschalter oder einer Lichtschranke erfaßt und die Antriebseinheit übt auf eines (12) der Aufnahmeelemente eine Kraft aus, so daß das Zugseil unter einer Vorspannung von beispielsweise 30 Newton oder 60 Newton gehalten wird, durch welche das Zangenmaul 3 in einer geschlossenen Grundposition gehalten wird. Der Abstand der beiden Nuten 7, 8 voneinander hängt von der Krümmung des Bowdenzuges 2 ab. Alternativ können statt der elektronisch geregelt zueinander verschiebbaren Aufnahmeelemente 11, 12 auch eine Mehrzahl vorderer Nuten 7 oder hinterer Nuten 8 am Griffbereich des Entnahmewerkzeugs 1 ausgebildet sein, wie in Figur 7 gezeigt ist. Die Ankopplung an die Aufnahmeelemente der Betätigungseinheit 10 erfolgt dann über diejenige der zirkulären Nuten 7, die in der jeweils besten Position befindlich ist.

Figur 6 zeigt schematisch die Betätigung des Zangenmauls 3 über die Bewegung der Aufnahmeelemente der Betätigungseinheit 10. In der in Figur 6 oben gezeigten Position haben die Nuten 7, 8 den kleinstmöglichen Abstand voneinander und das Zangenmaul 3 ist geöffnet. Der Griffteil 4 der Sonde weist eine in der Figur nicht dargestellte durchgehende Längsnut auf und ist fest mit der Nut (Nuten) 7 und der äußeren Hohlsonde bzw. Mantelsonde des Bowdenzuges 2 verbunden. Gegenüber dem Griffteil 4 längs verschieblich ist ein Verschiebeteil 9 mit zirkulärer Nut 8 ausgebildet. Das Verschiebeteil 9 weist einen (nicht gezeigten) Stift auf, der in die Längsnut des Griffteils 4 vorsteht und mit dem Zugseil des Bowdenzuges, das das Zangenmaul bewegt, verbunden ist. Eine Bewegung der Nut 8 nach vorne öffnet das Zangenmaul (Figur 6 oben, durch Pfeile angedeutet), während die entgegengesetzte Bewegung des Verschiebeteils 9 mit Nut 8 weg von der vorderen Nut 7 das Zangenmaul 3 schließt (Figur 6 unten). In der Vorspannungs-Grundposition ist das Zangenmaul mit einer Schließkraft von beispielsweise 10 - 60 Newton geschlossen. In einer zweiten Kraftstufe kann die Schließkraft zwischen 60 und 120 Newton oder mehr betragen.

Die (nicht gezeigte) Antriebseinheit zur Bewegung eines der Aufnahmeelemente 10, 11 und damit zur Betätigung des Greifmechanismus 3 kann vorzugsweise ein Elektromotor sein. Eine mit Druckluft oder hydraulisch betriebene Kraftquelle ist jedoch ebenfalls denkbar. Die Kraft kann von der separat angeordneten oder direkt mit der Betätigungseinheit 10 verbundenen Kraftquelle über einen Bowdenzug, über Zahnräder, über Gestänge und Kipphebel oder über Hydraulikleitungen auf das Aufnahmeelement übertragen werden. Die Steuerung des Antriebsmechanismus erfolgt vorzugsweise über ein Fußpedal, die dem Endoskopiker freie Hände für seine Tätigkeit läßt. Vorzugsweise ist das Entnahmewerkzeug 1 in Vorspannungs-Grundposition, wenn kein Pedal betätigt ist. Das Drücken eines Pedals bewirkt ein Öffnen des Zangenmauls und das Loslassen ein Schließen mit der normalen Vorspannung von 10 Newton bis 60 Newton und das Drücken eines zweiten Pedals das Öffnen des Zangenmauls und anschließendes Schließen mit einer Kraft von 60 Newton bis 120 Newton.

Figur 8 zeigt schematisch die auf einer stativartigen Halterung 22 drehbar gehaltene Betätigungseinheit 10 mit angekoppeltem Entnahmewerkzeug 1. An der Halterung 22 ist mit Abstand zur Betätigungseinheit 10 ein vorzugsweise entfernbares Führungsrohr 20, beispielsweise aus Kunststoff, befestigt, durch das das vordere Ende der Sonde 1 geführt werden kann. So kann der Endoskopiker nach durchgeführter Gewebeentnahme die Sonde aus dem Instrumentierkanal des Endoskops herausziehen und zwischenlagern, ohne daß eine assistierende Person ihm dieses abnehmen muß. Durch die Drehbarkeit der Betätigungseinheit 10 gegenüber der Halterung 22 und den Abstand und Winkel zwischen Betätigungseinheit 10 und Führungsrohr 20 kann die Sonde 1 eine Schlaufe bilden, wobei eine Berührung mit der Halterung oder auch anderen Gegenständen oder Personen wegen möglicher Kontamination vermieden wird. Zu diesem Zwecke kann auch ein Folienschlauch zwischen der Betätigungseinheit und dem Ventil des Endoskopkanals angebracht sein, der die Sonde 1 umgibt. Zusätzlich kann ein Schlauch mit einer Verzweigung am Endoskop angebracht sein, so daß ein Ende des Schlauchs mit einer Vorrichtung zur Aufnahme der entnommenen Gewebeprobe verbunden ist. Der Endoskopiker kann das vordere Ende der Sonde so innerhalb des Schlauchs aus dem Instrumentierkanal des Endoskops herausziehen und durch die Verzweigung der Probenaufnahmevorrichtung zuführen und die Probe dort versorgen. Die Verzweigung kann mit einem einstellbaren Ventil oder einem Element aus elastischem knickbaren Material versehen sein. Zur Erleichterung der Probenentfernung kann die Sonde, wie in Figur 9 gezeigt ist, mit einer fühlbaren oder sichtbaren Markierung 13 versehen sein, die einen definierten Abstand vom vorderen Ende des Greifmechanismus angibt. Die Markierung kann beispielsweise eine Farbmarkierung oder eine zirkuläre Nut oder ein zirkulärer Ring sein.

Im folgenden wird kurz die Handhabung der erfindungsgemäßen Vorrichtung zur endoskopischen Entnahme von Gewebeproben erläutert. Der Endoskopiker führt das Endoskop an die gewünschte Position beispielsweise im Magen-Darm-Trakt des menschlichen Körpers ein. Zur Gewebsentnahme (Biopsie) wird das Griffteil der Sonde 1 zunächst in die Aufnahmeelemente 11, 12 der Betätigungseinheit 10 eingerastet, so daß der Greifmechanismus 3 mit der eingestellten Vorspannung geschlossen ist. Die Sonde 1 mit daran befestigtem Greifmechanismus 3 kann dann leicht durch das Führungsrohr 20 und den Instrumentierkanal des Endoskops zu der gewünschten Probeentnahmestelle geführt werden. Dort betätigt der Endoskopiker ein Pedal, wodurch das hintere Aufnahmeelement 12 gegenüber dem vorderen Aufnahmeelement 11 verfahren wird, so daß sich das Zangenmaul 3 öffnet. Befindet sich die Gewebeprobe innerhalb des Zugriffsbereichs der Zange, wird diese durch Loslassen des Pedals mit einer eingestellten Kraft wieder geschlossen. Das Zangenmaul hält den entnommenen Gewebepartikel fest und die Sonde 1 kann wieder herausgezogen werden. Dabei bildet sich zwischen Führungsrohr 20 und der Betätigungseinheit 10 eine Schlaufe der Sonde, die frei in der Luft hängt und das Halterungsgestell der Vorrichtung nicht berührt. Die Drehbarkeit der Betätigungseinheit 10 verhindert ein Abknicken der Sonde und ermöglicht einen kurzen Abstand zwischen Führungsrohr 20 und Betätigungselement 10, der eine kurze Bauart der Biopsiezange ermöglicht. Die Gewichtskraft der Sondenschlaufe führt zu einem Reibschluß im Führungsrohr 20, so daß die Sonde im Führungsrohr fixiert ist und das Halterungsgestell 22 mit dem Entnahmewerkzeug durch den Endoskopiker vom Patienten zur späteren Versorgung weggeschoben werden kann. Eine assistierende Person ist somit während der endoskopischen Gewebeentnahme nicht mehr erforderlich. Außerdem läßt sich das Entnahmewerkzeug durch die automatische Vorspannung leicht handhaben und die Zugriffskraft bei der Probenentnahme einstellen.

## Patentansprüche

1. Vorrichtung zur Betätigung eines Greifmechanismus (3) an einer Sonde, vorzugsweise zur endoskopischen Entnahme von Gewebeproben oder Fremdkörpern, aufweisend
eine Hohlsonde (1) mit einem Greifmechanismus (3),
eine Betätigungseinheit (10) mit Aufnahmeelementen (11, 12) zur lösbaren Arretierung an einem Griffteil (4) der Sonde (1), und
eine Antriebseinheit in der Betätigungseinheit (10) zur kontrollierten Bewegung eines in der Hohlsonde (2) angeordneten Zugseiles zur Betätigung des Greifmechanismus (3),
**dadurch gekennzeichnet,**
daß das Griffteil (4) der Sonde (1) zwei Rastaufnahmen (7, 8) zum Einrasten in die Aufnahmeelemente (11, 12) der Betätigungseinheit (10) aufweist, wobei eines der Aufnahmeelemente (11, 12) mechanisch mit dem Zugseil gekoppelt ist,
daß die Aufnahmeelemente (11, 12) der Betätigungseinheit (10) zueinander beweglich sind, und
daß die Antriebseinheit ausgebildet ist, das mechanisch mit dem Zugseil der an der Betätigungseinheit (10) eingerasteten Hohlsonde (1) gekoppelte Aufnahmeelement (11, 12) zu bewegen.

2. Vorrichtung gemäß Anspruch 1, **dadurch gekennzeichnet,** daß die Rastaufnahmen als Nuten (7, 8) ausgebildet sind.

3. Vorrichtung gemäß Anspruch 2, **dadurch gekennzeichnet,** daß zur einrastenden Aufnahme eines der beiden Aufnahmeelemente (11, 12) mehrere in Abstand zueinander angeordnete zirkuläre Nuten (7) an dem Griffteil (4) der Sonde (1) ausgebildet sind.

4. Vorrichtung gemäß einem der Ansprüche 1 bis 3, **gekennzeichnet durch** einen Detektor in Form eines Druckschalters oder eine Lichtschranke zur Erfassung des in die Aufnahmeelemente (11, 12) eingerasteten Entnahmewerkzeugs (1).

5. Vorrichtung gemäß einem der Ansprüche 1 bis 4, **dadurch gekennzeichnet,** daß die Betätigungseinheit (10) elektronisch so gesteuert wird, daß beim Einlegen des Griffteils (4, 9) der Sonde (1) in eines der Aufnahmeelemente (10, 11) diese so nachgeführt werden, daß der Abstand der Aufnahmeelemente (11, 12) voneinander dem Abstand der Nuten (7, 8) des Griffteils (4, 9) der Sonde entspricht.

6. Vorrichtung gemäß einem der Ansprüche 4 oder 5, **dadurch gekennzeichnet,** daß die Betätigungseinheit (10), sobald die Sonde (1) in den Aufnahmeelementen (11, 12) eingerastet ist, eines der Aufnahmeelemente (11, 12) so verfährt, daß eine definierte Vorspannung zum Schließen des Greifmechanismus (3) auf das Zugseil (2) ausgeübt wird.

7. Vorrichtung nach einem der Ansprüche 4 bis 6, **dadurch gekennzeichnet,** daß die Zugriffskraft des Greifmechanismus (3) zwischen verschiedenen Werten einstellbar ist.

8. Vorrichtung gemäß einem der Ansprüche 1 bis 7, **dadurch gekennzeichnet,** daß die Betätigungseinheit (10) drehbar und gegebenenfalls höhenverstellbar an einer stativartigen Halterung (22) befestigt ist.

9. Vorrichtung gemäß Anspruch 8, **dadurch gekennzeichnet,** daß an der stativartigen Halterung (22) ein Führungsrohr (20) für die Sonde (1) vorgesehen ist.

10. Vorrichtung gemäß Anspruch 9, **dadurch gekennzeichnet,** daß das Führungsrohr (20) aus Kunststoff lösbar an der Halterung (22) angeordnet ist und als austauschbares Wegwerfteil ausgebildet ist.

11. Vorrichtung gemäß einem der Ansprüche 8 bis 10, **dadurch gekennzeichnet,** daß das Führungsrohr (20) mit einem Abstand und Winkel zur Betätigungseinheit (10) derart angeordnet ist, daß die Sonde (1) zwischen dem Griffteil (4) und dem Greifmechanismus (3) beim Zurückziehen des Greifmechanismus (3) im Bereich zwischen Betätigungseinheit (10) und Führungsrohr (20) eine Schlaufe bildet und eine Berührung mit anderen Teilen der Vorrichtung verhindert wird.

12. Vorrichtung gemäß einem der Ansprüche 8 bis 11, **dadurch gekennzeichnet,** daß zwischen Betätigungseinheit (10) und Führungsrohr (20) ein die Sonde (1) aufnehmender Folienschlauch oder Faltenschlauch zur Vermeidung einer Kontamination durch die Sonde (1) vorgesehen ist.
